# EUROPEAN PATENT SPECIFICATION

(11) **EP 0 503 128 B1**
(45) Date of publication and mention of the grant of the patent: **11.10.1995**
(21) Application number: 91110959.3
(22) Date of filing: 02.07.1991
(51) Int. Cl.: C12M 3/00, C12M 1/12, B01D 63/08

(54) **Apparatus for collection and transfer of particles and manufacture thereof**
Vorrichtung zur Sammlung und Übertragung von Partikeln und Verfahren zur Herstellung einer solchen Vorrichtung
Procédé pour la fabrication d'un appareil et appareil pour le prélèvement et le transfert de particules

(30) Priority: 13.03.1991 US 668892
(43) Date of publication of application: 16.09.1992
(73) Proprietor: CYTYC CORPORATION, Marlboro, Massachusetts 01752 (US)
(72) Inventor: Polk, Lewis T., Jr., Bedford, MA 01730 (US); Vartanian, Hugh, Methuen, MA 01844 (US)
(74) Representative: Blumbach, Kramer & Partner

(56) References cited:
- EP-A- 0 261 676
- EP-A- 0 448 837
- DE-A- 3 829 028
- DE-B- 2 541 000
- GB-A- 2 043 478

## Description

This invention relates to a device for collecting microscopic particles, such as cells, from a fluid medium and for transferring the collected cells to a receiver, such as a glass microscope slide. The device collects the cells with a desired spatial distribution, and enables the cells to be transferred to the receiver with that same spatial distribution.

A collection and transfer device according to the invention is useful in the performance of cytological procedures. In a typical cytological procedure, cells are collected from a liquid containing sample cells to be examined, and are transferred onto a microscope slide or other receiver for viewing and evaluation.

Cytological examination typically requires that the cells on the microscope slide be in a single layer and be separated from one another, i.e. dispersed. In some practices, the cells are counted, so that a generally known quantity of the cells is examined.

One prior device for collecting a dispersed monolayer of cells, and for transferring them to a microscope slide for examination, has a surface-trapping filter spanning an end of a tube. To collect cells, that end of the tube is immersed in a liquid that contains cells to be examined. A vacuum is applied to the tube to aspirate liquid through the filter. This flow of aspirated liquid into the tube deposits cells on the outer side of the filter. Each cell on the filter obstructs further flow of liquid through the filter, and therefore the cells collect on the filter with a generally dispersed and single layer spatial distribution. The filter is subsequently placed against the microscope slide to transfer the collected cells to the slide with that some spatial distribution. A slight fluid pressure is preferably applied to the inner side of the filter to facilitate this transfer of the cells to the slide. Also, it generally is desired to use the filter device only once and then discard it, to avoid inter-sample contamination. Accordingly, it is desired that the filter device be suited for high volume manufacture at relatively low cost.

The pending and commonly assigned European Patent Application No. 90125586.9 (EP-A-0 448 837, published on October 2, 1991) for "METHOD AND APPARATUS FOR CONTROLLED INSTRUMENTATION OF PARTICLES WITH A FILTER DEVICE" discloses a filter device of the above character for collecting cells from a sample liquid and with a desired spatial distribution. That application for patent, and the pending and commonly assigned European Patent Application No. 91109286.4 (EP-A-0 465 832, published on January 15, 1992) for "METHOD AND APPARATUS FOR PREPARING CELLS FOR EXAMINATION", disclose apparatus and procedures for performing cell collection with the filter device and for transferring the collected cells to a microscope slide with the desired dispersed spatial distribution.

It has been found that not all the cells collected with the known filter device of the type described above transfer to the viewing slide. Instead, different ones of the filter devices transfer different portions of the collected cells. This loss of cells is undesirable, and the variations in the portion of cells that are transferred is also undesirable. The loss of transferred cells reduces the number of cells available for inspection and it can result in a generally unknown quantity of cells being transferred to the glass slide.

It accordingly is an object of this invention to provide a device for the collection of cells and like particles from a fluid medium with a dispersed spatial distribution and for the transfer of the collected particles to a glass slide or like receiver with minimal loss of particles. It is also an object that the device facilitate transfer of the particles with faithful retention of the spatial distribution of the collected particles.

A further object is to provide a structure and manufacture for such a device so that different devices provide a consistent and uniform transfer of collected particles to the receiver.

Another object of the invention is to provide a particle collecting and transferring device of the above character that can be manufactured at relatively low cost. It is a corresponding object to provide a method for manufacturing such a device that can be practiced at relatively low cost.

Other objects of the invention will in part be obvious and will in part appear hereinafter.

### SUMMARY OF THE INVENTION

The present invention relates to an apparatus 10, 50 for particle collection and transfer, said apparatus comprising
A. a tubular support body 14, 56 having a first axial end spaced from a second axial end;
B. said body 14, 56 terminating, at said first axial end, in planar rim means 16, 60;
C. said support body 14, 56 defining annular attachment means 44 located on the periphery of said body 14, 56 intermediate said axial ends;
D. a surface-trapping filter element 12, 54 having a filter portion spanning said first end of said support body 14 and in abutment with said rim means 16, 60 for planar disposition thereof;
E. said filter element 12, 54 having a mounting portion 18, 62 peripheral to said first portion and secured to said annular attachment means 44 for attachment to said support body 14, 56; and
F. distortion-barrier means 30, 62 for isolating said rim means 16, 60 from distorting action generated in the course of bonding said mounting portion 18, 62 of said filter 12, 54 to said annular attachment means 44.

The invention also relates to a method for fabricating a particle-collecting device 10, 50, said method comprising the steps of
A. providing a tubular support body 14, 56 having a first axial end spaced from a second axial end;
B. providing rim means 16, 60 at said first end ad providing annular attachment means 44 on said body 14, 56 intermediate said axial ends;
C. providing annular trough means 34, 66 on said body 14, 56 adjacent to said attachment means 44 and between said attachment means 44 and said rim means 16, 60;
D. disposing a particle-collecting portion of a filter means 12, 54 on said rim means 16; 60; and
E. securing a peripheral securing portion of said filter means 12, 54 to said attachment means 44 and for attachment of said filter means 12, 54 to said body 14, 56.

A particle collecting and transferring device according to the invention can be used to transfer essentially all collected cells to a glass slide or other receiver, with faithful replication of the spatial distribution of the cells as collected. The device attains these objectives by disposing a filter of the surface-trapping type on a support body with a highly planar geometry. The filter collects the particles of interest on its outer surface. The planar disposition of the filter enables it to contact the receiver, such as a microscope slide, completely and uniformly, so that a consistent and high percentage of the collected particles transfer to the receiver. Further, the transferred particles have the same spatial distribution as they had on the filter prior to the transfer.

The invention stems from the finding that the prior structure and manufacture of the known filter device provided a surface-trapping filter that was not entirely flat, and from the finding that different ones of the device have filters that exhibit varying departures from flatness.

The invention thus provides a new structure for a device that collects particles such as cells from a fluid sample. The new structure enables the particle collecting device to transfer essentially all the collected particles to a microscope slide, and with a substantially exact replication of the spatial distribution that the cells had when on the collection device.

One embodiment of the new structure supports a sheet-like filter sheet, e.g. a pore membrane filter, on a planar tubular rim located at the end of a support body. The filter has a precise degree of flatness defined by the tubular rim. The new structure fastens the filter sheet to the support body at an annular surface recessed inwardly on the body below the rim. This new structure allows the filter to be attached to the support body with a low cost bonding technique, examples of which include thermal bonding, ultrasonic welding, and solvent bonding. Distortions of the mounting structure caused by the bonding technique are confined to the mounting surface, and do not detract from the desired precision flatness of the filter-positioning rim.

An essential feature of the filter device is the provision of a barrier between the filter positioning rim and the mounting surface. The barrier retards a bonding effect, such as heat, that is developed in the bonding process from disturbing the rim, where it might cause undesired geometrical distortion. The barrier can also be configured to receive excess or dislodged material produced during the bonding procedure, so that such material does not detract from the precision disposition of the filter on the rim. One embodiment of such a barrier structure is an annular trough on the support body located between the positioning rim and the mounting surface. The trough is geometrically disposed to have a depth dimension that increases the conduction path length between heat generated at the mounting surface and the positioning rim.

It is accordingly one feature of the invention to provide a particle collecting and transferring device that has a planar surface-trapping filter on a support body. The support body preferably is a tubular body that mounts the filter on a planar rim at one end. The body has an annular mounting surface disposed radially outward of the rim and axially inward on the body from the rim. The mounting surface preferably is generally parallel to the rim.

A further feature of the apparatus is a surface-trapping type filter having a first portion spanning the tubular body and seated on the rim for disposition in the plane defined by the rim. The filter first portion thus has an outer surface, facing outwardly from the tubular body, on which microscopic particles carried in a fluid can be collected, and from which such collected particles can be transferred to a receiver such as a microscope slide. The receiver is brought into abutment with the particle-carrying outer surface of the filter first portion to effect that transfer of the collected particles.

The features of the collecting and transferring device further include a mounting portion of the filter peripheral to the first portion and that is secured to the annular mounting surface, and thereby attached to the support body.

The structure for securing the mounting portion of the filter preferably includes a bond, between the material of the tubular body and the material of the filter. That bond typically is a substantially solid bonding flow of material produced with a technique such as ultrasonic welding, thermal bonding, or solvent bonding.

It is also a feature of the collecting and transferring device to provide a distortion barrier between the planar rim and the mounting surface. The barrier isolates the rim from material-distorting action developed during the attachment of the filter to the mounting surface. The barrier in a preferred embodiment is formed by an annular trough on the housing body and disposed between the rim and the mounting surface and having a depth dimension extending longitudinal to the axis of the tubular body.

Features of the invention further include a method of fabricating a particle collecting device as set forth above.

The foregoing and further features of the particle collecting and transferring device, and its manufacture, which the invention provides, attain a highly planar surface-trapping filter for the collection of particles from a fluid and for the precise uniform abutment with a microscope slide or other receiver, so that a consistent and maximal portion of the collected particles can readily be transferred to that receiver, and with a maximal retention of the spatial distribution that the particles had on the filter.

Further, the device can be manufactured at relatively low cost and in high volume. This is desirable to enable a fresh device to be used with each cytological sample being processed, and thereby to avoid intersample contamination.

The invention accordingly comprises the features of construction, combinations of elements and arrangements of parts exemplified in the constructions hereinafter set forth, and comprises the several steps and their relation of one or more of such steps with respect to each of the others for fabricating such a structure, all as exemplified in the following detailed disclosure, and the scope of the invention is indicated in the claims.

### BRIEF DESCRIPTION OF DRAWINGS

For a full understanding of the nature and objects of the invention, reference is to be made to the following detailed description and the accompanying drawings, in which:
FIGURE 1 is a perspective view of a collection and transfer device according to the invention;
FIGURE 2 is a side elevation view, partly in section, of the device of FIGURE 1;
FIGURE 3 is an enlarged fragmentary detail of FIGURE 2, without the viewing slide; and
FIGURE 4 is a fragmentary side elevatiion view, partly in section of another collection and transfer device according to the invention.

### DESCRIPTION OF ILLUSTRATED EMBODIMENTS

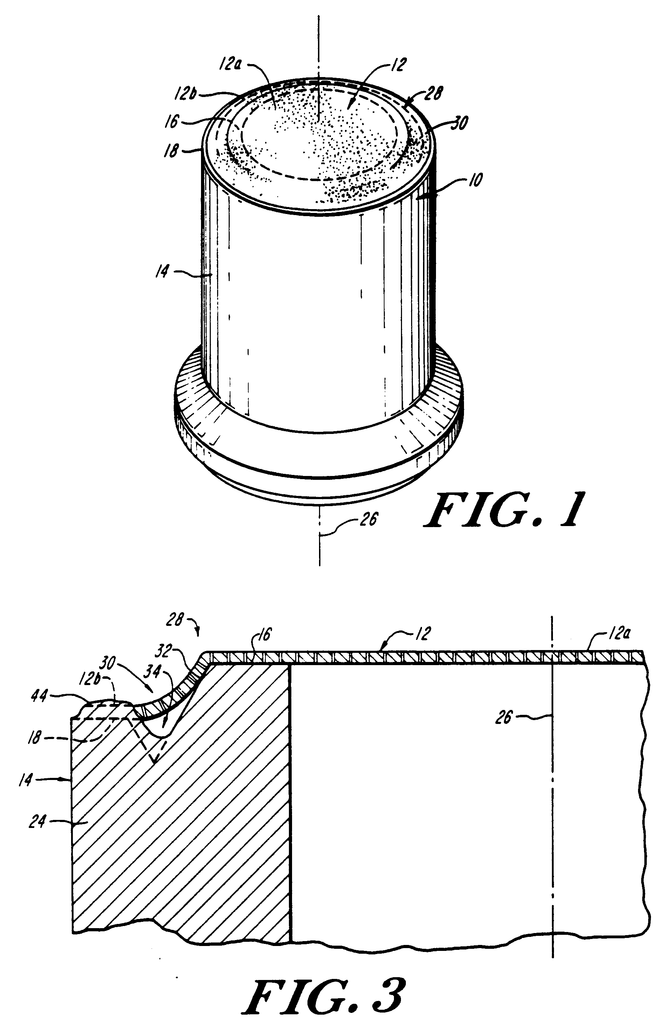

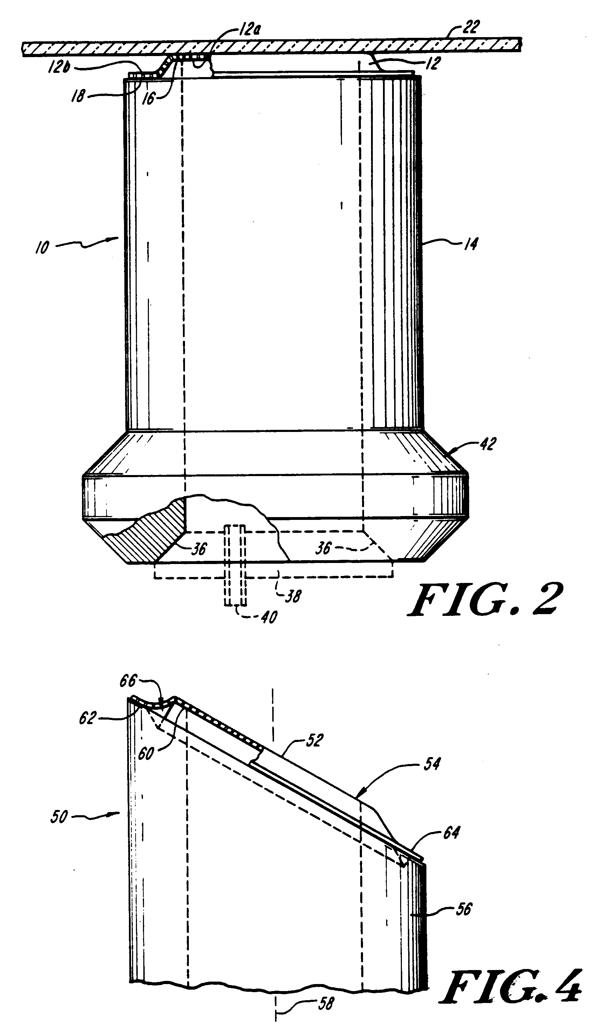

FIGURES 1 and 2 show a particle collecting and transfer device 10 according to the invention with a surface-trapping filter 12 mounted on a tubular body 14. The illustrated filter is a known pore membrane structure. A rim 16 at one end of the body 14 positions a central portion 12a of the filter in a flat configuration, i.e. planar, and the filter is secured at a peripheral portion 12b to a mounting surface 18 that is recessed inwardly on the body 14 from the rim 16.

The structure isolates the rim 16, so that it can position the filter with precision, from the location of the attachment of the filter to the body, i.e. from the mounting surface 18. Distortions of the mounting surface due to the mode of attachment thus do not alter the precise positioning geometry of the rim 16. As a result, the filter central portion 12a can have a precision planar disposition. When a glass microscope slide 22, shown in phantom in FIGURE 2, is brought into contact with the filter central portion 12a, essentially the entire filter central portion abuts the microscope slide. This substantially uniform abutment enables collected particles located throughout the entire surface of the filter central portion to be transferred directly to the microscope slide. There is essentially no loss of particles remaining on the filter, and the particles transfer with the same spatial distribution that they had on the filter.

More particularly, the illustrated tubular body 14 has a cylindrical wall 24 extending along an axis 26. The positioning rim 16 is at one axial end of the inner surface of the cylindrical wall 24. The annular end face 28 of the cylindrical wall 24 at that axial end, the inner portion of which forms the positioning rim 16, is axially recessed at a location radially outward of the positioning rim 16 to form the mounting surface 18. The illustrated mounting surface 18 is thus an annular surface concentric with the annular positioning rim 16 and is axially spaced from the rim inwardly on the body 14.

As appears in FIGURES 1 and 2 and further in the detail showing in FIGURE 3, a preferred yet generally optional structural feature of the device 10 is a barrier indicated generally at 30 on the body 14 and interposed between the positioning rim 16 and the mounting surface 18. The illustrated barrier 30 includes an annular trough 34 oriented so that the depth dimension extend longitudinal to the axial spacing between the rim 16 and the mounting surface 18. Correspondingly, the width of the illustrated barrier trough 34 extends in the radial direction, and is interposed radially between the rim 16, which is radially inward from the barrier, and the mounting surface 18, which is radially outward from the barrier.

The barrier 30 also includes the collar-like axially extending wall 32 that extends on the body 14 between the positioning rim 16 and the mounting surface 18.

The barrier 30, and particularly the trough 34, present a long thermal conduction path that isolates any heat developed at the mounting surface 18, during attachment of the filter thereto, from distorting or otherwise geometrically altering the positioning rim 16. This long path also retards solvent action, when solvent bonding is used, from deforming the rim 16. Further, the void formed by the barrier trough 34 provides a receptacle for trapping and otherwise receiving debris and other excess or flowing material produced during the bonding or other attachment of the filter peripheral portion 12b to the mounting surface 18. The barrier 30 thus substantially isolates the positioning rim from distortion and from material debris that might otherwise alter or otherwise detract from the desired highly planar surface which the positioning rim defines.

Further structure of the illustrated device 10 includes, adjacent its other axial end, a seat 36 that seals removably and replaceably with a cover plate 38 fitted with a through conduit 40. The cover plate can fit with the cylindrical wall 24 of the seat 36 to provide a fluid tight seal closing that end of the tubular body 14, except for the aperture which the conduit 40 provides.

As also shown in FIGURES 1 and 2, the illustrated device 10 has an outwardly projecting mounting and drive hub 42 disposed adjacent the sealing end of the body 14. As described further in the co-pending applications for patent noted above, the device 10 can be mounted in an instrument by way of the positioning hub 42 and rotated by way of idler rollers that engage the hub 42. The device 10 can, in addition, be sealed closed in the instrument by way of the cover plate 38, with the only openings being by way of the conduit 40 and the filter central portion 12a. When the device is inverted from the position shown in FIGURES 1 and 2 and the lower, filter-carrying end is immersed in a liquid sample containing particles, a vacuum applied to the conduit 40 aspirates liquid into the interior of the tubular body 14 and, in the process, deposits particles carried in the liquid onto the outer surface of the filter central portion 12a.

As further described in the noted co-pending applications for patent, the device 10 with particles on the outer surface of the filter central portion 12a, after removal from the particle containing liquid, can be placed in abutment with a microscope slide 22, as illustrated in FIGURE 3. The cells or other particles collected on the filter are transferred to the glass slide. The transfer of particles from the filter to the microscope slide can be facilitated by applying an elevated pressure to the inner side of the filter portion 12a.

With further reference to FIGURES 1, 2, and 3, the filter 12 is secured to the tubular body 14 by way of attachment of the peripheral portion 12b to the mounting surface 18. A preferred attachment is obtained by ultrasonically welding the filter peripheral portion to the material of the tubular body at the mounting surface 18. With this fabrication process, after the filter 12 is positioned on the rim 16 of the cylindrical wall 24 of the tubular body 14, an ultrasonic weld horn is brought into selected pressure contact with the filter peripheral portion 12b, pressing it against the mounting surface 18. The ultrasonic energy radiated by the ultrasonic welding horn creates a bonding flow of material of the tubular body 14 that bonds the filter peripheral portion to the mounting surface. The resultant bond 44, FIGURE 3, preferably is both mechanical and intermolecular, upon solidification of the flowed material.

Alternative techniques for securing the filter 12 to the mounting surface 18 include thermal bonding and solvent bonding. These techniques, including ultrasonic welding, are generally known in the art.

However, with a preferred practice of the invention using a tubular body 14 in which the cylindrical wall 24 is molding from polystyrene resin marketed by the Dow Chemical Company under the designation Styron^{R} 685D, and with a filter 12 that is a polycarbonate membrane treated with a wetting agent, as commercially available from manufacturers to be hydrophilic, the ultrasonic bonding can be carried out using a process available from Polyfiltronics, Inc. of Rockland, Massachusetts 02370, U.S.A. The porous membrane that forms the filter 12 in this example is in the order of 7 µm (seven microns) thick, and is available commercially from Poretics Corporation, Livermore, California 94550, U.S.A, and from Nuclepore Corporation, Pleasonton, California 94566, U.S.A..

As noted above, material distorting affects developed at the mounting surface 18 during this securing of the filter peripheral portion to the mounting surface, including for example heat and solvent action, are isolated from the positioning rim 16 by way of the barrier 30 and including the trough 34. Further, material that becomes fluid or otherwise dislodged during the mounting process --such as material of the filter and/or of the tubular body cylindrical wall --, and which tends to move radially inwardly on the annular mounting surface 18, is trapped or otherwise received in the barrier trough 34. The trough thus prevents such material from being deposited on, or otherwise distorting or disfiguring, the positioning rim 16. The barrier 30 thus ensures that the rim 16 positions the filter central portion 12a with the desired shape or contour, e.g., planar flatness.

FIGURE 4 shows another device 50 according to the invention and having an oval-shaped portion 52 of a filter 54 for collecting particles and for transferring them to a receiver such as a viewing slide. The device 50 has a tubular support body 56, elongated along an axis 58, and forms an eliptical filter-positioning rim 60. The rim is flat, i.e. planar, and is in a plane that extends diagonally across the circular cross-section of the illustrated body 56.

Axially-spaced inwardly on the tubular body 56 from the rim 60 is a mounting shoulder 62 that projects radially outward on the tubular outer wall of the body 56. The shoulder forms an annular mounting surface to which a peripheral portion 64 of the filter 54 attaches, for attaching the filter to the body. The illustrated mounting surface is oval-shaped, i.e. eliptical, and is preferably generally parallel to the positioning rim 60.

The device 50 also has a barrier trough 66 that axially recesses the mounting surface formed by the shoulder 62. The trough is at a radially inner location on the shoulder, adjacent the tubular wall of the body 56.

The filter 54 is attached to the body 56 by securing the peripheral portion 64 to the mounting surface formed by the shoulder 62. The method of securing can employ a technique such as ultrasonic welding, thermal bonding or solvent bonding. The further structure and method of manufacture for the device 50 can be in accord with the preceding description, including the description of the device 10 shown in FIGURES 1-3.

It will thus be seen that the invention efficiently obtains the objects set forth above, among those made apparent from the previous description. Since certain changes may be made in carrying out the above process or in the device set forth without departing from the scope of the invention, it is intended that all matter contained in the above description or shown in the accompanying drawing be interpreted as illustrative and not in a limiting sense.

It is also to be understood that the following claims are to cover all generic and specific features of the invention described herein, and all statements of the scope of the invention which, as a matter of language, might be said to fall there between.

## Claims

1. Apparatus (10; 50) for particle collection and transfer, said apparatus comprising
A. a tubular support body (14; 56) having a first axial end spaced from a second axial end;
B. said body (14; 56) terminating, at said first axial end, in planar rim means (16; 60);
C. said support body (14; 56) defining annular attachment means (44) located on the periphery of said body (14; 56) intermediate said axial ends;
D. a surface-trapping filter element (12; 54) having a filter portion spanning said first end of said support body (14; 56) and in abutment with said rim means (16; 60) for planar disposition thereof;
E. said filter element (12; 54) having a mounting portion (18; 62) peripheral to said first portion and secured to said annular attachment means (44) for attachment to said support body (14; 56); and
F. barrier means (30; 66) for isolating said rim means (16; 60) from distorting action generated in the course of bonding said mounting portion (18; 62) of said filter (12; 54) to said annular attachment means (44).

2. Apparatus according to claim 1 wherein said mounting portion (18; 62) of said filter element (12; 54) is secured to said annular attachment means (44) by a substantial solid bonding between the material of said support body (14, 56) and the material of said filter element (12, 54).

3. Apparatus according to claim 1, wherein said barrier means (30; 66) comprises a trough (34; 66) for receiving debris generated in bonding said mounting portion (18; 62) of said filter (12; 54) to said attachment means (44), said trough being located between said attachment means (44) and said rim means (16; 60).

4. Apparatus according to one or more of the preceding claims wherein said rim means (16; 60) comprises a annular filter-supporting surface conforming to a first plane and extending transversely relative to the axis (26; 58) of said body (14; 56).

5. Apparatus according to one or more of the preceding claims wherein said mounting portion (18; 62) of said filter element (12; 54) is either ultrasonically welded or thermally bonded to said attachment means (44).

6. A method for fabricating a particle-collecting device (10; 50), said method comprising the steps of
A. providing a tubular support body (14; 56) having a first axial end spaced from a second axial end;
B. providing rim means (16; 60) at said first end and providing annular attachment means (44) on said body (14; 56) intermediate said axial ends;
C. providing annular trough means (34; 66) on said body (14; 56) adjacent to said attachment means (44) and between said attachment means (44) and said rim means (16; 60);
D. disposing a particle-collecting portion of a filter means (12; 54) on said rim means (16; 60); and
E. securing a peripheral securing portion of said filter means (12; 54) to said attachment means (44) for attachment of said filter means (12; 54) to said body (14; 56).

7. Method according to claim 6 wherein wherein said securing step comprises a securing process selected from ultrasonic welding, thermal bonding and solvent bonding.

8. Method according to claims 6 and 7 wherein said securing step produces heat at said attachment means (44) and further comprises the step of isolating said rim means (16; 60) from deformation by such heat.

9. Apparatus having a particle-collecting and transferring filter (12; 54), said apparatus comprising
A. a tubular body (14; 56) having a planar rim (16; 60) at a first end;
B. an annular mounting surface (18; 62) on said body (14; 56) disposed radially outwardly of said rim (16; 60) and generally parallel thereto, said mounting surface (18; 62) being spaced axially inward on said body (14; 56) from said rim (16; 60);
C. a sheet-like surface-trapping filter (12; 54) having a filter portion spanning said tubular body (14; 56) and seated on said rim (16; 60) for disposition in the plane defined by said rim (16; 60), said filter portion having a outer surface facing outwardly from said tubular body (14; 56) for collecting thereon microscopic particles carried in a fluid and for the transfer of such collected particles to a planar receiver brought into abutment with said particle-carrying outer surface;
D. a mounting portion (18; 62) of said filter peripheral to said filter portion;
E. means for forming a bond between said mounting surface (18; 62) of said body (14; 56) and said mounting portion of said filter for securing said filter (12; 54) to said body (14; 56); and
F. barrier means (30; 66) on said body (14; 56) disposed between said mounting surface (18; 62) and said rim (16; 60).

10. Apparatus according to claim 9 further comprising means for forming a thermal conduction barrier disposed between said mounting surface (18; 62) and said rim (16; 60) for isolating said rim (16; 60) from material-distorting action developed at said mounting surface (18; 62) when forming said filter-securing bond.

11. Apparatus according to claims 9 and 10 further comprising a barrier means (30) arranged on said body (14; 56) as an annular trough means 34; 66) disposed between said mounting surface (18; 62) and said rim (16; 60) and having a depth dimension extending parallel to the axis (26, 58) of said body (14; 56).

12. Apparatus according to any of the claims 1 to 5 and 9 to 11 further comprising a drive hub means (42) projecting outwardly from said tubular body (14; 56) for receiving a mounting force applied to one axial end of the tubular body (14; 56).

## Patentansprüche

1. Vorrichtung (10; 50) zum Sammeln und Übertragen von Teilchen, umfassend
A. einen rohrförmigen Halter-Körper (14; 56) mit einem ersten axialen Ende, das von einem zweiten axialen Ende entfernt ist;
B. wobei der Körper (14; 56) an dem ersten axialen Ende in einen planaren Rand (16; 60) endet;
C. wobei der Halter-Körper (14; 56) eine ringförmige Befestigungseinrichtung (44) definiert, die auf dem Außenumfang des Körpers (14; 56) zwischen den axialen Enden angeordnet ist;
D. ein an der Oberfläche abfangendes Filterelement (12; 54), das einen Filter-Abschnitt aufweist, der das erste Ende des Halter-Körpers (14; 56) überspannt und zu seiner planaren Anordnung in Anlage mit dem Rand (16; 60) ist;
E. wobei das Filterelement (12; 54) einen Montageabschnitt (18; 62) aufweist, der peripher zu dem ersten Abschnitt angeordnet und an der ringförmigen Befestigungseinrichtung (44) zur Befestigung an dem Halter-Körper (14; 56) befestigt ist; und
F. eine Barriere-Einrichtung (30; 66), um den Rand (16; 60) vor einer Deformation zu bewahren, die im Verlauf des Bindens des Montageabschnitts (18; 62) des Filters (12; 54) an die ringförmige Befestigungseinrichtung (44) auftritt.

2. Vorrichtung nach Anspruch 1, worin der Montageabschnitt (18; 62) des Filterelements (12; 54) an der ringförmigen Befestigungseinrichtung (44) mittels einer im wesentlichen festen Verbindung zwischen dem Material des Halter-Körpers (14; 56) und dem Material des Filterelements (12; 54) befestigt ist.

3. Vorrichtung nach Anspruch 1, worin die Barriere-Einrichtung (30; 66) eine Rinne (34; 66) zur Aufnahme von Trümmern umfaßt, die beim Binden des Montage-Abschnitts (18; 62) des Filters (12; 54) an die Befestigungseinrichtung (44) erzeugt werden, wobei die Rinne zwischen der Befestigungseinrichtung (44) und dem Rand (16; 60) angeordnet ist.

4. Vorrichtung nach einem oder mehreren der vorangehenden Ansprüche, worin der Rand (16; 60) eine ringförmige, das Filter tragende Oberfläche umfaßt, die sich an eine erste Ebene anpaßt und sich quer zur Achse (26; 58) des Körpers (14; 56) erstreckt.

5. Vorrichtung nach einem oder mehreren der vorangehenden Ansprüche, worin der Montageabschnitt (18; 62) des Filterelements (12; 54) mit der Befestigungseinrichtung (44) entweder ultraschall-verschweißt oder an diese thermisch gebunden wird.

6. Verfahren zur Herstellung einer Teilchen-Sammelvorrichtung (10; 50), wobei das Verfahren die Schritte umfaßt, daß man
A. einen rohrförmigen Halter-Körper (14; 56) schafft, der ein erstes axiales Ende aufweist, das von einem zweiten axialen Ende entfernt ist;
B. einen Rand (16; 60) an dem ersten Ende ausbildet und eine ringförmige Befestigungseinrichtung (44) an dem Körper (14; 56) ausbildet, die zwischen den axialen Enden liegt;
C. eine ringförmige Rinneneinrichtung (34; 66) an dem Körper (14; 56) vorsieht, die der Befestigungseinrichtung (44) benachbart ist und zwischen der Befestigungseinrichtung (44) und dem Rand (16; 60) liegt;
D. einen Abschnitt einer Filtereinrichtung (12; 54) zum Sammeln von Teilchen auf dem Rand (16; 60) anordnet; und
E. einen peripheren Sicherungsabschnitt der Filtereinrichtung (12; 54) an der Befestigungseinrichtung (44) zur Befestigung der Filtereinrichtung (12; 54) an dem Körper (14; 56) befestigt.

7. Verfahren nach Anspruch 6, worin der Schritt des Befestigens ein Befestigungsverfahren umfaßt, das gewählt ist aus Ultraschall-Schweißen, thermischem Verbinden und Lösungsmittel-Verbinden.

8. Verfahren nach den Ansprüchen 6 und 7, worin der Befestigungsschritt Wärme an der Befestigungseinrichtung (44) erzeugt und außerdem den Schritt umfaßt, daß man den Rand (16; 60) gegen eine Verformung durch diese Wärme isoliert.

9. Vorrichtung mit einem Filter (12; 54) zum Sammeln und Übertragen von Teilchen, umfassend
A. einen rohrförmigen Körper (14; 56) mit einem planaren Rand (16; 60) an einem ersten Ende;
B. eine ringförmige Montagefläche (18; 62) an dem Körper (14; 56), die radial außerhalb des Randes (16; 60) und allgemein parallel dazu angeordnet ist, wobei die Montagefläche (18; 62) von dem Rand (16; 60) axial nach innen auf dem Körper (14; 56) beabstandet ist;
C. ein einer dünnen Schicht ähnliches, an der Oberfläche abfangendes Filter (12; 54), das einen Filter-Abschnitt aufweist, der den rohrförmigen Körper (14; 56) überspannt und auf dem Rand (16; 60) zur Anordnung in der durch den Rand (16; 60) definierten Ebene sitzt, wobei der Filter-Abschnitt eine Außenfläche, die von dem rohrförmigen Körper (14; 65) nach außen zeigt, zum Sammeln mikroskopischer Teilchen, die in einer Flüssigkeit zugegen sind, und zur Übertragung derartiger gesammelter Teilchen auf eine planare Aufnahme-Einrichtung aufweist, die in Anlage mit der teilchentragenden Außenfläche gebracht wird;
D. einen Montageabschnitt (18; 62) des Filters peripher zu dem Filter-Abschnitt;
E. eine Einrichtung zum Ausbilden einer Bindung zwischen der Montagefläche (18; 62) des Körpers (14; 56) und dem Montageabschnitt des Filters zur Befestigung des Filters (12; 54) an dem Körper (14; 56); und
F. einer Barriere-Einrichtung (30; 66) an dem Körper (14; 56), die zwischen der Montagefläche (18; 62) und dem Rand (16; 60) angeordnet ist.

10. Vorrichtung nach Anspruch 9, umfassend außerdem eine Einrichtung zur Bildung einer Barriere gegen thermische Leitung, die zwischen der Montagefläche (18; 62) und dem Rand (16; 60) angeordnet ist, um den Rand (16; 60) gegenüber einer das Material deformierenden Wirkung zu isolieren, die an der Montagefläche (18; 62) entwickelt wird, wenn die das Filter befestigende Bindung gebildet wird.

11. Vorrichtung nach den Ansprüchen 9 und 10, umfassend außerdem eine Barriere-Einrichtung (30), die an dem Körper (14; 56) als ringförmige Rinnen-Einrichtung (34; 66) angeordnet und zwischen der Montagefläche (18; 62) und dem Rand (16; 60) angeordnet ist und eine Tiefendimension aufweist, die sich parallel zur Achse (26; 58) des Körpers (14; 56) erstreckt.

12. Vorrichtung nach einem der Ansprüche 1 bis 5 und 9 bis 11, außerdem umfassend eine Antriebsnaben-Einrichtung (42), die von dem rohrförmigen Körper (14; 56) nach außen vorsteht, um eine Montage-Kraft aufzunehmen, die auf ein axiales Ende des rohrförmigen Körpers (14; 56) aufgebracht wird.

## Revendications

1. Appareil (10; 50) pour la prélèvement et le transfert de particules, ledit appareil comprenant
A. un corps formant support tubulaire (14; 56) ayant une première extrémité axiale espacée d'une deuxième extrémité axiale ;
B. ledit corps (14; 56) se terminant, à ladite première extrémité axiale, en des moyens formant collerette plane (16; 60) ;
C. ledit corps formant support (14; 56) définissant des moyens de fixation annulaires (44) placés sur la périphérie dudit corps (14; 56) entre lesdites extrémités axiales ;
D. un élément formant filtre (12; 54) à piège en surface ayant une partie filtrante recouvrant ladite première extrémité dudit corps formant support (14; 56) et venant en butée contre lesdits moyens formant collerette (16; 60) pour une disposition plane de celle-ci.
E. ledit élément filtrant (12; 54) ayant une partie de montage (18; 62) à la périphérie de ladite première partie et fixé auxdits moyens de fixation annulaires (44) pour être fixé audit corps formant support (14; 56) ; et
F. des moyens formant barrière (30; 66) destinés à isoler lesdits moyens formant collerette (16; 60) d'une action déformante générée au cours de la liaison de ladite partie du montage (18; 62) dudit filtre (12; 54) auxdits moyens de fixation annulaires (44).

2. Appareil selon la revendication 1, dans lequel ladite partie de montage (18; 62) dudit élément filtrant (12; 54) est fixée auxdits moyens de fixation annulaires (44) par une liaison substantiellement solide entre le matériau dudit corps formant support (14; 56) et le matériau dudit élément filtrant (12; 54).

3. Appareil selon la revendication 1, dans lequel lesdits moyens formant barrière (30; 66) comprennent une cuvette (34; 66) destinée à recevoir des débris produits lors de la liaison de ladite partie de montage (18; 62) dudit filtre (12; 54) auxdits moyens de fixation (44), ladite cuvette étant placée entre lesdits moyens de fixation (44) et lesdits moyens formant collerette (16; 60).

4. Appareil selon une ou plus des revendications précédentes, dans lequel lesdits moyens formant collerette (16; 60) comprennent une surface formant support de filtre annulaire s'adaptant à un premier plan et s'étendant transversalement par rapport à l'axe (26; 58) dudit corps (14; 56).

5. Appareil selon une ou plus des revendications précédentes, dans lequel ladite partie de montage (18; 62) dudit élément filtrant (12; 54) est soit soudée par ultrasons soit thermocollée auxdits moyens de fixation (44).

6. Procédé pour fabriquer un appareil pour le prélèvement de particules (10; 50), ledit procédé comprenant les étapes consistant à :
A. fournir un corps formant support tubulaire (14; 56) ayant une première extrémité axiale espacée d'une deuxième extrémité axiale ;
B. fournir des moyens formant collerette (16; 60) à ladite première extrémité et fournir des moyens de fixation annulaires (44) sur ledit corps (14; 56) entre lesdites extrémités axiales ;
C. fournir des moyens formant cuvette (34; 66) sur ledit corps (14; 56) adjacents auxdits moyens de fixation (44) et entre lesdits moyens de fixation (44) et lesdits moyens formant collerette (16; 60) ;
D. placer une partie de moyens filtrants (12; 54) pour le prélèvement de particules sur lesdits moyens formant collerette (16; 60) ; et
E. fixer une partie de fixation périphérique desdits moyens filtrants (12; 54) auxdits moyens de fixation (44) pour fixer lesdits moyens filtrants (12; 54) audit corps (14; 56).

7. Procédé selon la revendication 6, dans lequel ladite étape de fixation comprend un procédé de fixation choisi parmi le soudage par ultrasons, le thermocollage et le collage au solvant.

8. Procédé selon les revendications 6 et 7, dans lequel ladite étape de fixation produit de la chaleur auxdits moyens de fixation (44) et comprend en outre l'étape d'isolation desdits moyens formant collerette (16; 60) de la déformation par une telle chaleur.

9. Appareil ayant un filtre (12; 54) pour le prélèvement et le transfert de particules, ledit appareil comprenant
A. un corps tubulaire (14; 56) ayant une collerette plane (16; 60) à une première extrémité ;
B. une surface de montage (18; 62) annulaire sur ledit corps (14; 56) placée radialement à l'extérieur de ladite collerette (16; 60) et généralement parallèle à celle-ci, ladite surface de montage (18; 62) étant espacée axialement à l'intérieur sur ledit corps (14; 56) depuis ladite collerette (16; 60) ;
C. un filtre en forme de feuille (12; 54) formant piège en surface ayant une partie filtrante recouvrant ledit corps tubulaire (14; 56) et reposant sur ladite collerette (16; 60) pour une disposition dans le plan défini par ladite collerette (16; 60), ladite partie filtrante ayant une surface extérieure tournée vers l'extérieur depuis ledit corps tubulaire (14; 56) pour prélever sur celle-ci des particules microscopiques transportées dans un fluide et pour transférer de telles particules prélevées jusqu'à un récepteur plan mis en butée contre ladite surface extérieure portant les particules ;
D. une partie de montage (18; 62) dudit filtre à la périphérie de ladite partie filtrante ;
E. des moyens pour former une liaison entre ladite surface de montage (18; 62) dudit corps (14; 56) et ladite partie de montage dudit filtre pour fixer ledit filtre (12; 54) audit corps (14; 56) ; et
F. des moyens formant barrière (30; 66) sur ledit corps (14; 56) placés entre ladite surface de montage (18; 62) et ladite collerette (16; 60).

10. Appareil selon la revendication 9 comprenant en outre des moyens pour former une barrière de transfert de chaleur placée entre ladite surface de montage (18; 62) et ladite collerette (16; 60) pour isoler ladite collerette (16; 60) d'une action de déformation de matériau développée sur ladite surface de montage (18; 62) lorsque se forme la liaison de fixation du filtre.

11. Appareil selon les revendications 9 et 10 comprenant en outre des moyens formant barrière (30) agencés sur ledit corps (14; 56) comme moyens formant cuvette annulaire (34, 66) placée entre ladite surface de montage (18; 62) et ladite collerette (16; 60) et ayant une dimension en profondeur s'étendant parallèlement à l'axe (26; 58) dudit corps (14; 56).

12. Appareil selon l'une quelconque des revendications 1 à 5 et 9 à 11 comprenant en outre des moyens d'actionnement de moyeu (42) faisant saillie à l'extérieur depuis ledit corps tubulaire (14; 56) destinés à recevoir une force de montage appliquée à une extrémité axiale du corps tubulaire (14; 56).
